# EUROPEAN PATENT APPLICATION

(11) **EP 3 698 812 A1**
(43) Date of publication of application: **26.08.2020**
(21) Application number: 18900288.4
(22) Date of filing: 09.03.2018
(51) Int. Cl.: A61K 47/69, A61K 47/32, A61K 47/10, A61K 47/12, A61K 47/02, A61K 9/08, A61K 9/19, A61K 31/337, A61P 35/00, A61P 35/04

(54) **CABAZITAXEL COMPOSITION FOR INJECTION AND PREPARATION METHOD THEREFOR**

(30) Priority: 11.01.2018 CN 201810025193
(71) Applicant: Bika Biotechnology (Guangzhou) Co., Ltd., Guangzhou, Guangdong 510310 (CN)
(72) Inventor: TONG, Keqin, Nanjing, Jiangsu 210038 (CN); CHENG, Jinlan, Nanjing, Jiangsu 210038 (CN)
(74) Representative: Schiweck Weinzierl Koch Patentanwälte Partnerschaft mbB
(86) International application number: PCT/CN2018/078567
(87) International publication number: WO 2019/136817

(57) **Abstract**

The present invention relates to a cabazitaxel composition for injection and a preparation method therefor. The composition comprises the following components in parts by weight: 1 part of cabazitaxel, 1 to 100 parts of cyclodextrin, 10 to 200 parts of a cosolvent, 1 to 60 parts of polyvidone (PVP), and 0.02 to 1.0 part of an additive. The composition does not contain polysorbate and ethanol. The composition of the present invention contains no polysorbate and ethanol, has low histamine release, and does not require the use of antihistamines, corticosteroids and H₂ antagonists before administration. The composition is provided in a single ready-to-use vial, and serves as a new composition preparation that does not require two-step dilution. The preparation has characteristics of cabazitaxel, including high solubility and high stability. The preparation remains stable long after redissolution and is convenient to use clinically. Since the composition does not contain polysorbate and ethanol, side effects such as an allergic reaction, skin irritation, or addiction are reduced. Further disclosed is a preparation method of the cabazitaxel composition for injection.

## Description

### CROSS-REFERENCE TO RELATED APPLICATION

This application claims the benefit of Chinese patent application No. 201810025193.4, filed on January 11, 2018, which is hereby incorporated by reference in its entirety.

### FIELD OF INVENTION

The present invention relates to the field of pharmaceutical preparation, more particularly to a cabazitaxel composition for injection and preparation method therefor.

### BACKGROUND ART

Cabazitaxel is a white or off-white powder, almost insoluble in water, soluble in ethanol, and unstable under alkaline conditions. Its chemical name is (1S,2S,3R,4S,7R,9S,10S,12R,15S)-4-acetyloxy-15-{[(2R,3S)-3-{[(tertbutoxy)carbonylamino]}-2-hydroxy-3-phenylpropanoyl]oxy}-1-hydroxy-9, 12-dimethoxy-10,14,17,17-tetramethyl-11-oxo-6-oxatetracyclo[11.3.1.0^{3;10}.0^{4;7}] heptadec-13-ene-2-yl benzoate. Its molecular formula is C₄₅H₅₇N₁₄, its molecular weight is 835.93 and its structure is shown as below:

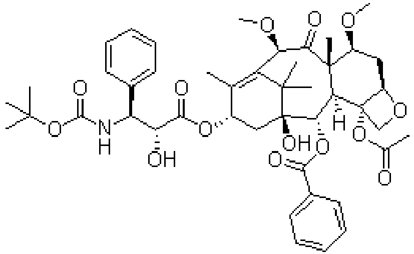

Cabazitaxel is a new generation of taxane anti-tumor drugs, which may be prepared by semi-synthesis of precursors extracted from yew, it resembles docetaxel in anti-cancer mechanism and characteristics, and belongs to anti-microtubule drugs. Cabazitaxel promotes its assembly into microtubules by binding with tubulin. At the same time, it can prevent the disintegration of the assembled microtubules, stabilize the microtubules, and then inhibit the mitosis of cells and the function of interphase cells. Compared with Taxol and docetaxel, cabazitaxel has a stronger ability to inhibit tumor cell proliferation and is effective in patients with docetaxel-resistant tumors. In 2010, the U.S. FDA approved the cabazitaxel preparation JEVTANA® developed by Sanofi-aventis for the treatment of the patients with hormone-refractory metastatic prostate cancer. It is the first therapeutic drug approved by FDA for the prostate cancer which are resistant to docetaxel, and it can significantly prolong the survival of patients with advanced prostate cancer.

Cabazitaxel has strong lipophilicity and is almost insoluble in water. Its commercial preparation JEVTANA® employs surfactant polysorbate 80 (Tween 80) as a solubilizer and ethanol as a diluent. The preparation package contains two vials: (a) JEVTANA® injection, 60mg cabazitaxel in 1.5mL polysorbate 80; (b) diluent, about 5.7mL of 13%(w/w) ethanol solution. This preparation requires a two-step preparation process before being administered to patients: in the first step, the (a) JEVTANA ® injection is mixed with the (b) diluent to form a mixture with a concentration of about 10mg/mL; in the second step, the mixture prepared in the first step is diluted into a 250mL container (non-PVC) containing 0.9% sodium chloride solution or 5% dextrose solution for injection. The concentration of cabazitaxel in the final infusion solution should be 0.10 mg/mL ∼ 0.26 mg/mL.

Polysorbate (Tween) is a non-ionic surfactant. It has a foul odor, is warm and slightly bitter. It is a series of partial fatty acid esters of polyoxyethylene sorbitan. It is widely used as an emulsifier and a solubilizer for oils. Polysorbate is generally considered to be a non-toxic and non-irritant material. It is often used as a solubilizing excipient for injectable poorly soluble drugs, however because of the side effects from Tween by injection administration, for example, hemolysis can occur at low concentration (0.01∼2.0%), allergic reactions occur during topical and intramuscular administration, in severe cases, systemic rash / erythema, hypotension and / or bronchospasm or very rare fatal allergic reactions or even death will occur. In order to reduce the side effects of polysorbate, it is necessary for patients to administrate antihistamine, corticosteroid and H₂ antagonist clinically, which greatly reduces the compliance of clinical use.

The presence of polysorbate 80 in JEVTANA® can cause severe side effects, such reactions have been reported in patients characterized by systemic rash / erythema, hypotension and / or bronchospasm or very rare fatal allergic reactions. In order to reduce the side effects induced by polysorbate 80, antihistamine, corticosteroid and H₂ antagonist should be administrated 30 minutes before intravenous administration of JEVTANA®. At the same time, JEVTANA ® needs to be diluted in two steps before clinical use: firstly diluted with 13% (w/w) ethanol solution, and then diluted with 0.9% sodium chloride solution or 5% glucose solution before administration, such use steps are complicated, and have potential dangers in medication. In addition, JEVTANA® uses a 13% (w/w) ethanol solution as a diluent, however, ethanol can cause side effects such as considerable irritation and addiction when administrated by injection.

Accordingly, there is a need for a new cebazitaxel preparation in clinical applications that has low side effects, does not require precursor administration and is simple, safe and convenient to use.

Cyclodextrin, a pharmaceutical excipient, has a hollow hydrophobic, stereoscopic chiral inner cavity; due to its structural characteristic of " inner hydrophobic, external hydrophilic", it can encapsulate a variety of small organic molecules (substrates) having suitable space size to form a noncovalent host-guest complex (inclusion complex). Its most significant pharmaceutical function is to increase the water solubility of poorly soluble drugs and improve the stability of drugs. For example, the invention application No. CA2900508A1 (US20150325321A1) by Valery Alakhov et al. disclosed a composition comprising cabazitaxel and suifobutyl ether beta cyclodextrin (SBE-β-CD) in a weight ratio of 1:30 to 1:1000. The experimental results show that the solubility of cabazitaxel in 40% SBE-β-CD aqueous solution is 4.17mg/mL. However, when the inventors of the present invention experimentally studied the solubilizing effect of SBE-β-CD on cabazitaxel, it was found that the solubility of cabazitaxel in 40% SBE-β-CD aqueous solution was about 1mg/mL, which cannot reach 4.17mg/mL as described in the invention application No. CA2900508A1. Even if it can reach such concentration, it is difficult to prepare a cabazitaxel injection that meets the concentration specifications of commercial preparations. For example, the JEVTANA® specification is 60 mg/1.5 mL, the solubility of cabazitaxel in 40% SBE-β-CD aqueous solution is about 1mg / mL, 60 mg cabazitaxel needs 60mL of such solution to dissolve. If the solubility of cabazitaxel is 4.17 mg/mL, 60 mg cabazitaxel needs 15 mL of such solution to dissolve, which is much different from 1.5mL per vial of JEVTANA® kit, it is difficult to industrialize in terms of preparation process, cost, packaging, transportation and storage, etc. At the same time, it is necessary to consider whether the cabazitaxel injection is stable and does not precipitate crystals or precipitates when diluted with the infusion solution such as 0.9% sodium chloride solution or 5% glucose solution to the infusion concentration range.

Based on the technical teaching that the commercially available preparation JEVTANA® containing polysorbate and ethanol, and the preparation in CA2900508A1 (US20150325321A1) containing a high proportion (greater than 1:30) of cyclodextrin, it is obvious for the skilled in the art to continuously increase the proportion of cyclodextrin and/or increase the amount of ethanol in order to substitute the preparation containing polysorbate. However, as a result of this attempt by the inventors, no matter whether the content of cyclodextrin or the amount of ethanol is increased, the solubility of cabazitaxel cannot be improved to meet the needs of clinical medicine.

### Summary of the Invention

In view of various defects of the prior art, the inventor of the present application breaks through the prejudice of the prior art, conducts reverse experiments on existing knowledge, reduces the amount of cyclodextrin and abandons the use of ethanol having strong stimulation as a solvent and Tween 80 having side effects. Specifically, the inventor of the present application adds additives for injection, such as citric acid, sodium bisulfite, and edetate disodium, etc., to a solution system comprising 700 mg/ml PEG300, 290 mg/ml SBE-β-CD, 80 mg/ml PVPK12 and an appropriate amount of water (about 100mg), then fills an appropriate amount of nitrogen, which turns out that the obtained composition can not only make the solubility of cabazitaxel ≥ 40 mg/ml, but also greatly improve the stability of cabazitaxel in the system.

In other words, based on the reverse experiments carried out by the inventor of the present application, a non-obvious or even unexpected result is obtained: a new composition consisting of cabazitaxel, cyclodextrin, polyethylene glycol (PEG), polyvinylketone (PVP) and additives for injection is obtained, this composition does not contain polysorbate and ethanol, can significantly improve the property of the cabazitaxel preparation, and can effectively increase the solubility and stability of cabazitaxel.

Research shows that the composition consisting of cabazitaxel, cyclodextrin, PEG and PVP without polysorbate and ethanol can not only overcome the side effects caused by the existing commercial preparations containing polysorbate, but also greatly reduce the injection irritation and addiction of the preparations and greatly improve the stability of the cabazitaxel preparation. The present technical solution overcomes the defect existing in the patent No. CA2900508A1, in which a large amount of cyclodextrin is used, but the solubility of cabazitaxel is not significantly improved, the solubility of cabazitaxel can only be increased up to 4.17mg/mL. Furthermore, the present technical solution overcomes the problem that the cabazitaxel composition in CA2900508A1 is not very stable and difficult to meet the practical problems of clinical use.

Therefore, the objective of the present invention is to develop a cabazitaxel for injection, which does not contain polysorbate and ethanol, is a single-vial ready-to-use, and does not require two-step dilution, the cabazitaxel for injection is stable, and meet the requirement of clinical medication, the safety and effectiveness.

### Definition

Single-vial ready-to-use refers to a sterile liquid or lyophilisate in a single vial, which can be administrated to patients intravenously after dilution or dissolution with an infusion solution to a clinical application concentration range, without the need for two-step dilution.

Infusion solution refers to a sterile isotonic solution, such as 0.9% sodium chloride solution or 5% glucose solution, which is usually stored in a bag or bottle and can be administered to patients after diluting or dissolving the preparation for injection.

### Technical Solution

To achieve the above-mentioned objective, the invention adopts the following technical solution:
In one aspect, the invention provides a carbataxel composition for injection, comprising the following components by weight part: 1 part of carbataxel, 1 ∼ 100 parts of cyclodextrin, 10 ∼ 200 parts of solubilizer, 1 ∼ 60 parts of polyvidone (PVP), 0.02 ∼ 1.0 parts of additive without containing polysorbate (such as Tween 80) and ethanol.

Preferably, the composition comprises the following components by weight part: 1 part of carbataxel, 10 - 30 parts of cyclodextrin, 30 - 150 parts of solubilizer, 1 ∼ 15 parts of polyvidone (PVP), 0.05 - 0.8 parts of additive without containing polysorbate and ethanol.

More preferably, the composition comprises the following components by weight part: 1 part of carbataxel, 25 - 29 parts of cyclodextrin, 50 - 90 parts of solubilizer, 7 ∼ 15 parts of polyvidone (PVP), 0.05 - 0.8 parts of additive without containing polysorbate and ethanol.

Further preferably, the composition comprises the following components by weight part: 1 part of carbataxel, 26 - 29 parts of cyclodextrin, 60 - 80 parts of solubilizer, 7 ∼ 10 parts of polyvidone (PVP), 0.1 - 0.7 parts of additive without containing polysorbate and ethanol.

Preferably, the cyclodextrin includes but not limited to suifobutyl ether beta cyclodextrin (SBE-β-CD), hydroxypropyl beta cyclodextrin (HP-β-CD) and/or hydroxypropyl suifobutyl ether beta cyclodextrin (HP-SBE-β-CD); preferably SBE-β-CD.

Preferably, the solubilizer is one or more selected from but not limited to polyethylene glycol (PEG), propylene glycol and glycerin, preferably polyethylene glycol (PEG).

Preferably, the polyethylene glycol (PEG) is one or more selected from but not limited to PEG200, PEG300, PEG400, PEG600, PEG800, PEG1000, PEG1500 and PEG2000, preferably PEG300 and/or PEG400.

Preferably, the polyvidone (PVP) is one or more selected from but not limited to PVPK12, PVPK15, PVPK17, PVPK25, PVPK30, PVPK45, PVPK60, PVPK70, PVPK80, PVPK85, PVPK90, PVPK100, PVPK110, PVPK120 and PVPK150, preferably PVPK12 and/or PVPK17.

Preferably, the additive includes but not limited to citric acid and/or tartaric acid; and/or acetic acid; and/or hydrochloric acid; and/or phosphoric acid; and/or lactic acid; and/or ascorbic acid; and/or L-cysteine; and/or sodium bisulfite; and/or sodium pyrosulfite; and/or disodium edetate, preferably citric acid and/or sodium bisulfite.

Preferably, the composition is in the form of a solid lyophilisate or an aqueous solution suitable for storage.

In another aspect, the present invention provides a method for preparing the above-mentioned carbataxel composition for injection (carbataxel solid lyophilisate for injection) comprising the following steps:
(1) weighing the cyclodextrin, adding the solubilizer and water, adding the polyvidone and the additive after stirring to dissolve, stirring to dissolve, and then adding cabazitaxel and filling with inert gas (such as nitrogen) for protection, continuing to stir for 30 - 240min after stirring to dissolve, and obtaining an uniform mixed solution;
(2) taking samples to determine pH value and concentration, after qualification, filtering through 0.2 µm polytetrafluoroethylene (PTFE) membrane, sub-packing in vials, half-plugging, freeze-drying in a freeze dryer, filling nitrogen gas, plugging, sealing and labeling.

Preferably, in the step (1), the pH value of the uniform mixed solution is 2.0 - 6.0.

In a further aspect, the present invention provides another method for preparing the above-mentioned carbataxel composition for injection (carbataxel solid lyophilisate for injection) comprising the following steps:
(1) weighing the cyclodextrin, adding the solubilizer and water, adding the polyvidone and the additive after stirring to dissolve, stirring to dissolve, and then adding cabazitaxel and filling with inert gas (such as nitrogen) for protection, continuing to stir for 30 - 240min after stirring to dissolve, and obtaining an uniform mixed solution;
(2) taking samples to determine pH value and concentration, after qualification, filtering through 0.2 µm polytetrafluoroethylene (PTFE) membrane, sub-packing in vials, filling nitrogen, plugging, sealing and labeling.

Preferably, in the step (1), the pH value of the uniform mixed solution is 2.0 - 6.0.

In still another aspect, the present invention provides a method for treating tumors, comprising administering the above composition or the composition prepared by the above method to a patient in need; preferably, the tumor is prostate cancer, which is hormone refractory metastatic prostate cancer or prostate cancer resistant to docetaxel, and this method can significantly prolong the survival time of the patients with advanced prostate cancer. The stability, the related substances and the stabilization time after re-dissolution of the cabazitaxel for injection prepared by the above technical solution are better than that in the existing commercial preparations by adopting the above technical solution due to the synergistic effect of the dissolution of solubilizer, the inclusion and solubilization of cyclodextrin, and the prevention of crystallization and growth of polyvidone, the appropriate pH adjusted by the additives and low-oxygen or near-hypoxic environment formed by low-concentration metal ions and nitrogen.

Therefore, the composition of the present invention does not contain polysorbate (e.g. Tween 80) and ethanol, has low histamine release and does not need to use antihistamines, corticosteroids and H₂ antagonists before administration. This composition is a single vial ready to use and a new preparation, which does not require two-step dilution. This preparation has the following features: high solubility of cabazitaxel, high stability and long stabilization time after re-dissolution and convenience for clinical application. The side effects such as allergy, irritation and addiction are reduced due to the absence of polysorbate and ethanol. Meanwhile, the present invention also provides a method for preparing the carbataxel composition for injection.

### DETAILED DESCRIPTION OF THE EMBODIMENTS

The present invention will be further described in detail in combination with the specific examples and are not intended to limit the scope of the invention.

### EXAMPLE 1

| | |
|---|---|
| Cabazitaxel: | 1.0g |
| SBE-β-CD: | 29g |
| PEG300: | 70g |
| PVPK12: | 8.0g |
| Water: | 10g |
| Citric acid: | 300mg |
| Sodium bisulfite: | 400mg |
| Prepared into | 100 vials |

A prescription amount of SBE-β-CD was weighed, a prescription amount of PEG300 and water were added, the agitation is performed at 23 ∼ 25 °C to dissolve, a prescription amounts of PVPK12 and citric acid were added, a prescription amount of sodium bisulfite and cabazitaxel were added after agitation to dissolve, the agitation was kept for 2h to form an uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 2

| | |
|---|---|
| Cabazitaxel: | 1.0g |
| SBE-β-CD: | 29g |
| PEG300: | 70g |
| PVPK12: | 8.0g |
| Water: | 10g |
| Citric acid: | 300mg |
| Prepared into | 100 vials |

A prescription amount of SBE-β-CD was weighed, a prescription amount of PEG300 and water were added, the agitation was performed at 22 ∼ 25°C to dissolve, a prescription amount of PVPK12 and citric acid were added, a prescription amount of cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 3

| | |
|---|---|
| Cabazitaxel: | 1.0g |
| SBE-β-CD: | 40g |
| PEG300: | 50g |
| PVPK12: | 8.0g |
| Water: | 12g |
| Citric acid: | 300mg |
| Prepared into | 100 vials |

A prescription amount of SBE-β-CD was weighed, a prescription amount of PEG300 and water were added, the agitation was performed at 18 ∼ 20°C to dissolve, a prescription amount of PVPK12 and citric acid were added, a prescription amount of cabazitaxel was added after agitation to dissolve, the agitation was kept for 100min to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 4

| | |
|---|---|
| Cabazitaxel: | 1.0g |
| SBE-β-CD: | 40g |
| PEG300: | 50g |
| PVPK12: | 8.0g |
| Water: | 12g |
| Citric acid: | 300mg |
| Prepared into | 100 vials |

A prescription amount of SBE-β-CD was weighed, a prescription amount of PEG300 and water were added, the agitation was performed at 20 ∼ 22°C to dissolve, a prescription amount of PVPK12 and citric acid were added, a prescription amount of cabazitaxel was added after agitation to dissolve, the agitation was kept for 240min to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, half-plugged, freeze-dried in a freeze-dryer, plugged, capped and labeled.

### EXAMPLE 5

| | |
|---|---|
| Cabazitaxel: | 1.0g |
| SBE-β-CD: | 29g |
| PEG400: | 70g |
| PVPK12: | 8.0g |
| Water: | 10g |
| Citric acid: | 300mg |
| Sodium bisulfite: | 400mg |
| Prepared into | 100 vials |

A prescription amount of SBE-β-CD was weighed, a prescription amount of PEG400 and water were added, the agitation was performed at 21 ∼ 23°C to dissolve, a prescription amount of PVPK12 and citric acid were added, a prescription amount of cabazitaxel was added after agitation to dissolve, the agitation was kept for 30min to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 6

| | |
|---|---|
| Cabazitaxel: | 1.0g |
| SBE-β-CD: | 29g |
| PEG400: | 70g |
| PVPK12: | 8.0g |
| Water: | 10g |
| Citric acid: | 300mg |
| Prepared into | 100 vials |

A prescription amount of SBE-β-CD was weighed, a prescription amount of PEG400 and water were added, the agitation was performed at 19 ∼ 22°Cto dissolve, a prescription amount of PVPK12 and citric acid were added, a prescription amount of cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 7

| | |
|---|---|
| Cabazitaxel: | 1.0g |
| HP-β-CD: | 40g |
| PEG300: | 57g |
| PVPK12: | 8.0g |
| Water: | 12g |
| Citric acid: | 300mg |
| Prepared into | 100 vials |

A prescription amount of HP-β-CD was weighed, a prescription amount of PEG300 and water were added, the agitation was performed at 18 ∼ 20°C to dissolve, a prescription amount of PVPK12 and citric acid were added, a prescription amount of cabazitaxel was added after agitation to dissolve, the agitation was kept for 180min to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 8

| | |
|---|---|
| Cabazitaxel: | 1.0g |
| HP-β-CD: | 40g |
| PEG300: | 57g |
| PVPK12: | 8.0g |
| Water: | 12g |
| Citric acid: | 300mg |
| Sodium bisulfite: | 400mg |
| Prepared into | 100 vials |

A prescription amount of HP-β-CD was weighed, a prescription amount of PEG300 and water were added, the agitation was performed at 18 ∼ 20°C to dissolve, a prescription amount of PVPK12 and citric acid were added, a prescription amount of sodium bisulfite and cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 9

| | |
|---|---|
| Cabazitaxel: | 1.0g |
| HP-SBE-β-CD: | 45g |
| PEG300: | 49g |
| PVPK12: | 8.0g |
| Water: | 15g |
| Citric acid: | 300mg |
| Prepared into | 100 vials |

A prescription amount of HP-SBE-β-CD was weighed, a prescription amount of PEG300 and water were added, the agitation was performed at 23 ∼ 25 °C to dissolve, a prescription amount of PVPK12 and citric acid were added, a prescription amount of cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 10

| | |
|---|---|
| Cabazitaxel: | 1.0g |
| HP-SBE-β-CD: | 45g |
| PEG300: | 49g |
| PVPK12: | 8.0g |
| Water: | 15g |
| Citric acid: | 300mg |
| Prepared into | 100 vials |

A prescription amount of HP-SBE-β-CD was weighed, a prescription amount of PEG300 and water were added, the agitation was performed at 23 ∼ 25 °C to dissolve, a prescription amount of PVPK12 and citric acid were added, a prescription amount of cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, half-plugged, free-dried in a free dryer, plugged, capped and labeled.

### EXAMPLE 11

| | |
|---|---|
| Cabazitaxel: | 1.0g |
| SBE-β-CD: | 19g |
| PEG300: | 75g |
| PVPK12: | 8.0g |
| Water: | 15g |
| Citric acid: | 300mg |
| Sodium bisulfite: | 400mg |
| Prepared into | 100 vials |

A prescription amount of SBE-β-CD was weighed, a prescription amount of PEG300 and water were added, the agitation was performed at 23 ∼ 25°Cto dissolve, a prescription amount of PVPK12 and citric acid were added, a prescription amount of sodium bisulfite and cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 12

| | |
|---|---|
| Cabazitaxel: | 1.0g |
| SBE-β-CD: | 10g |
| PEG300: | 70 |
| PVPK12: | 8.0g |
| Water:: | 16g |
| Citric acid: | 300mg |
| Sodium bisulfite: | 400mg |
| Prepared into | 100 vials |

A prescription amount of SBE-β-CD was weighed, a prescription amount of PEG300 and water were added, the agitation was performed at 23 ∼ 25°Cto dissolve, a prescription amount of PVPK12 and citric acid were added, a prescription amount of sodium bisulfite and cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 13

| | |
|---|---|
| Cabazitaxel: | 1.0g |
| SBE-β-CD: | 29g |
| Propylene glycol: | 70g |
| PVPK12: | 8.0g |
| Water: | 10g |
| Citric acid: | 300mg |
| Sodium bisulfite: | 400mg |
| Prepared into | 100 vials |

A prescription amount of SBE-β-CD was weighed, a prescription amount of propylene glycol and water were added, the agitation was performed at 23 ∼ 25°C to dissolve, a prescription amount of PVPK12 and citric acid were added, a prescription amount of sodium bisulfite and cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 14

| | |
|---|---|
| Cabazitaxel: | 1.0g |
| SBE-β-CD: | 29g |
| Glycerol: | 70g |
| PVPK12: | 8.0g |
| Water: | 10g |
| Citric acid: | 300mg |
| Sodium bisulfite: | 400mg |
| Prepared into | 100 vials |

A prescription amount of SBE-β-CD was weighed, a prescription amount of propylene glycol and water were added, the agitation was performed at 23 ∼ 25°C to dissolve, a prescription amount of PVPK12 and citric acid were added, a prescription amount of sodium bisulfite and cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 15

| | |
|---|---|
| Cabazitaxel: | 1.0g |
| SBE-β-CD: | 29g |
| PEG300: | 70g |
| PVPK12: | 4.0g |
| Water: | 10g |
| Citric acid: | 300mg |
| Sodium bisulfite: | 400mg |
| Prepared into | 100 vials |

A prescription amount of SBE-β-CD was weighed, a prescription amount of PEG300 and water were added, the agitation was performed at 23 ∼ 25°Cto dissolve, a prescription amount of PVPK12 and citric acid were added, a prescription amount of sodium bisulfite and cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXAMPLE 16

| | |
|---|---|
| Cabazitaxel: | 1.0g |
| SBE-β-CD: | 29g |
| PEG300: | 70g |
| PVPK12: | 20g |
| Water: | 10g |
| Citric acid: | 300mg |
| Sodium bisulfite: | 400mg |
| Prepared into | 100 vials |

A prescription amount of SBE-β-CD was weighed, a prescription amount of PEG300 and water were added, the agitation was performed at 23 ∼ 25°Cto dissolve, a prescription amount of PVPK12 and citric acid were added, a prescription amount of sodium bisulfite and cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### COMPARATIVE EXAMPLE 1

| | |
|---|---|
| Cabazitaxel: | 1.0g |
| SBE-β-CD: | 29g |
| PVPK12: | 8.0g |
| Water: | 80g |
| Citric acid: | 300mg |
| Sodium bisulfite: | 400mg |
| Prepared into | 100 vials |

A prescription amount of SBE-β-CD was weighed, a prescription amount of water were added, the agitation was performed at 23 ∼ 25°C to dissolve, a prescription amount of PVPK12, citric acid and sodium bisulfite was added, a prescription amount of cabazitaxel was added after agitation to dissolve, the agitation was performed, but cabazitaxel was not completely dissolved.

### COMPARATIVE EXAMPLE 2

| | |
|---|---|
| Cabazitaxel: | 1.0g |
| SBE-β-CD: | 29g |
| PEG300: | 70g |
| Water: | 18g |
| Citric acid: | 300mg |
| Sodium bisulfite: | 400mg |
| Prepared into | 100 vials |

A prescription amount of SBE-β-CD was weighed, a prescription amount of PEG300 and water were added, the agitation was performed at 23 ∼ 25°C to dissolve, a prescription amount of citric acid were added, a prescription amount of sodium bisulfite and cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### COMPARATIVE EXAMPLE 3

| | |
|---|---|
| Cabazitaxel: | 1.0g |
| SBE-β-CD: | 29g |
| Water: | 88g |
| Citric acid: | 300mg |
| Sodium bisulfite: | 400mg |
| Prepared into | 100 vials |

A prescription amount of SBE-β-CD was weighed, a prescription amount of water were added, the agitation was performed at 23 ∼ 25°C to dissolve, a prescription amount of citric acid and sodium bisulfite was added, a prescription amount of cabazitaxel was added after agitation to dissolve, the agitation was performed, but cabazitaxel was not completely dissolved.

### COMPARATIVE EXAMPLE 4

| | |
|---|---|
| Cabazitaxel: | 1.0g |
| PEG300: | 70g |
| PVPK12: | 8.0g |
| Water:: | 39g |
| Citric acid: | 300mg |
| Sodium bisulfite: | 400mg |
| Prepared into | 100 vials |

A prescription amount of PEG300 and water were added, the agitation was performed at 23 ∼ 25°C to dissolve, a prescription amount of PVPK12 and citric acid were added, a prescription amount of sodium bisulfite and cabazitaxel was added after agitation to dissolve, the agitation was kept for 2h to form a uniform solution after agitation to dissolve. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### COMPARATIVE EXAMPLE 5

| | |
|---|---|
| Cabazitaxel: | 1.0g |
| SBE-β-CD: | 29g |
| PEG300: | 205g |
| PVPK12: | 8.0g |
| Water: | 10g |
| Citric acid: | 300mg |
| Sodium bisulfite: | 400mg |
| Prepared into | 100 vials |

A prescription amount of SBE-β-CD was weighed, a prescription amount of PEG300 and water were added, the agitation was performed at 23 ∼ 25°C to dissolve, the materials were not completely dissolved, a prescription amount of PVPK12 and citric acid were added, the agitation was performed, a prescription amount of sodium bisulfite and Cabazitaxel were added, the agitation was kept for 2h. The solution was filtered through 0.2 µm polytetrafluoroethylene (PTFE) membrane, sub-packed in vials, filled with nitrogen, plugged, capped and labeled.

### COMPARATIVE EXAMPLE 6

| | |
|---|---|
| Cabazitaxel: | 1.0g |
| SBE-β-CD: | 29g |
| PEG300: | 70g |
| PVPK12: | 70g |
| Water: | 10g |
| Citric acid: | 300mg |
| Sodium bisulfite: | 400mg |
| Prepared into | 100 vials |

A prescription amount of SBE-β-CD was weighed, a prescription amount of PEG300 and water were added, the agitation was performed at 23 ∼ 25°Cto dissolve, a prescription amount of PVPK12 and citric acid were added, the materials were not completely dissolved, a prescription amount of sodium bisulfite and Cabazitaxel were added, the agitation was kept for 2h. The samples were taken to determine the pH value and the concentration, after qualification, the solution was filtered through 0.2 µm polytetrafluoroethylene (PTFE) membrane, sub-packed in vials, filled with nitrogen, plugged, capped and labeled.

### EXPERIMENTAL EXAMPLE

### 1. Solubility and re-dissolution experiments

According to the proportion of each substances in respective examples, the blank preparations without API (cabazitaxel) were prepared, and then excessive amount of API was added respectively, stirred for 12h at room temperature and in dark, filtered and diluted, and then the solubility was determined; at the same time, the re-dissolution of the preparations according to the examples and comparative examples was investigated. The results of the solubility and the re-dissolution were as follow:

| Items | API solubility (mg/mL) | Re-dissolution | Stabilization time after re-dissolution | Note |
|---|---|---|---|---|
| EXAMPLE 1 | 41.50 | Clear solution | Crystals precipitated in about 8h | |
| EXAMPLE 2 | 40.94 | Clear solution | Crystals precipitated in about 8h | |
| EXAMPLE 3 | 30.37 | Clear solution | Crystals precipitated in about 8h | |
| EXAMPLE 4 | 30.05 | Clear solution | Crystals precipitated in about 8h | |
| EXAMPLE 5 | 53.22 | Clear solution | Crystals precipitated in about 8h | |
| EXAMPLE 6 | 53.07 | Clear solution | Crystals precipitated in about 8h | |
| EXAMPLE 7 | 44.16 | Clear solution | Crystals precipitated in about 4h | |
| EXAMPLE 8 | 43.88 | Clear solution | Crystals precipitated in about 4h | |
| EXAMPLE 9 | 27.24 | Clear solution | Crystals precipitated in about 6h | |
| EXAMPLE 10 | 26.44 | Clear solution | Crystals precipitated in about 6h | |
| EXAMPLE 11 | 43.29 | Clear solution | Crystals precipitated in about 3h | |
| EXAMPLE 12 | 55.12 | Clear solution | Crystals precipitated in about 1h | |
| EXAMPLE 13 | 38.99 | Clear solution | Crystals precipitated in about 7h | |
| EXAMPLE 14 | 35.42 | Clear solution | Crystals precipitated in about 7h | |
| EXAMPLE 15 | 41.28 | Small amount of floccule | Crystals precipitated in about 7h | |
| EXAMPLE 16 | 41.56 | Clear solution | Crystals precipitated in about 8h | |
| | | | | |
| COMPARATIVE EXAMPLE 1 | 0.66 | / | / | Because API was not dissolved fully, the preparation cannot be obtained, there was no re-dissolution test. |
| COMPARATIVE EXAMPLE 2 | 40.64 | Small amount of floccule | Crystals precipitated in about 6h | |
| COMPARATIVE EXAMPLE 3 | 0.65 | / | / | Because API was not dissolved fully, the preparation cannot be obtained, there was no re-dissolution test. |
| COMPARATIVE EXAMPLE 4 | 67.09 | Crystallization | / | |
| COMPARATIVE EXAMPLE 5 | 67.22 | Small amount of floccule | Crystals precipitated in about 30min | SBE-β-CD was not fully dissolved |
| COMPARATIVE EXAMPLE 6 | 40.20 | Small amount of floccule | Crystals precipitated in about 8h | PVP was not fully dissolved |

### 2. Study on the amount of cyclodextrin

It can be seen from the comparison of the results of "EXAMPLE 1 and COMPARATIVE EXAMPLE 4" and the results of "EXAMPLE 1 and EXAMPLE 12" in the above Table that when the amount of cyclodextrin is within the preferred range of the present invention (such as 29g SBE-β-CD in EXAMPLE 1), the preparation is clear and can form a clear solution after redissolution, the stabilization time is about 8h, and then crystal precipitates; when the preparation does not contain cyclodextrin (there is no any cyclodextrin in COMPARATIVE EXAMPLE 4) or contains lower amount of cyclodextrin than the preferred range of the present invention (lOg SBE-β-CD in EXAMPLE 12), the formed preparation precipitate the crystals at about 1h when re-dissolution.

It can be seen from the comparison of the results of EXAMPLE 2 and EXAMPLE 3 that when the amount of cyclodextrin is within the preferred range of the present invention (29g SBE-β-CD in EXAMPLE 2) and the amount of cyclodextrin exceeds the preferred range of the present invention (40g SBE-β-CD in EXAMPLE 3), the obtained preparation are clear and a clear solution after re-dissolution, the stabilization time is about 8h, and then crystal precipitates, there is no obvious difference between the two prescriptions. However, considering the safety of pharmaceutical preparations, the amount of medicinal excipients or additives should be as small as possible, if the prescription in which the amount of cyclodextrin is in the preferred range and that in which the amount of cyclodextrin is higher than the preferred range exhibit the same effect, or if there is no obvious effect by increasing the amount, then it is better to choose the amount of cyclodextrin in the preferred range.

In conclusion, the stabilization time after re-dissolution for the preparation containing low amount of cyclodextrin is quite short, while there is no obvious effect improvement for the preparation containing high amount of cyclodextrin, therefore the amount in the preferred range is more appropriate.

## Claims

1. A carbataxel composition for injection, comprising the following components by weight part: 1 part of carbataxel, 1 - 100 parts of cyclodextrin, 10 - 200 parts of solubilizer, 1 ∼ 60 parts of polyvidone (PVP), 0.02 - 1.0 parts of additive without containing polysorbate and ethanol.

2. The composition according to claim 1, wherein the composition comprises the following components by weight part: 1 part of carbataxel, 10 ∼ 30 parts of cyclodextrin, 30 - 150 parts of solubilizer, 1 ∼ 15 parts of polyvidone (PVP), 0.05 - 0.8 parts of additive without containing polysorbate and ethanol.

3. The composition according to claim 1 or 2, wherein the composition comprises the following components by weight part: 1 part of carbataxel, 25 - 29 parts of cyclodextrin, 50 - 90 parts of solubilizer, 7 ∼ 15 parts of polyvidone (PVP), 0.05 - 0.8 parts of additive without containing polysorbate and ethanol;
preferably, the composition comprises the following components by weight part: 1 part of carbataxel, 26 - 29 parts of cyclodextrin, 60 - 80 parts of solubilizer, 7 ∼ 10 parts of polyvidone (PVP), 0.1 - 0.7 parts of additive without containing polysorbate and ethanol.

4. The composition according to any one of claims 1 to 3, wherein the cyclodextrin includes but not limited to suifobutyl ether beta cyclodextrin (SBE-β-CD), hydroxypropyl beta cyclodextrin (HP-β-CD) and/or hydroxypropyl suifobutyl ether beta cyclodextrin (HP-SBE-β-CD); preferably SBE-β-CD.

5. The composition according to any one of claims 1 to 4, wherein the solubilizer is one or more selected from but not limited to polyethylene glycol (PEG), propylene glycol and glycerin, preferably polyethylene glycol (PEG);
preferably, the polyethylene glycol (PEG) is one or more selected from but not limited to PEG200, PEG300, PEG400, PEG600, PEG800, PEG1000, PEG1500 and PEG2000, preferably PEG300 and/or PEG400.

6. The composition according to any one of claims 1 to 5, wherein the polyvidone (PVP) is one or more selected from but not limited to PVPK12, PVPK15, PVPK17, PVPK25, PVPK30, PVPK45, PVPK60, PVPK70, PVPK80, PVPK85, PVPK90, PVPK100, PVPK110, PVPK120 and PVPK150, preferably PVPK12 and/or PVPK17.

7. The composition according to any one of claims 1 to 6, wherein the additive includes but not limited to citric acid and/or tartaric acid; and/or acetic acid; and/or hydrochloric acid; and/or phosphoric acid; and/or lactic acid; and/or ascorbic acid; and/or L-cysteine; and/or sodium bisulfite; and/or sodium pyrosulfite; and/or disodium edetate, preferably citric acid and/or sodium bisulfite.

8. The composition according to any one of claims 1 to 7, wherein the composition is in the form of a solid lyophilisate or an aqueous solution suitable for storage.

9. A method for preparing a carbataxel composition for injection comprising the following steps:
(1) weighing the cyclodextrin, adding the solubilizer and water, adding the polyvidone and the additive after stirring to dissolve, stirring to dissolve, and then adding cabazitaxel and filling with inert gas (such as nitrogen) for protection, continuing to stir for 30 - 240min after stirring to dissolve, and obtaining an uniform mixed solution;
(2) taking samples to determine pH value and concentration, after qualification, filtering through 0.2 µm polytetrafluoroethylene (PTFE) membrane, sub-packing in vials, half-plugging, freeze-drying in a freeze dryer, filling nitrogen gas, plugging, sealing and labeling;
preferably, in the step (1), the pH value of the uniform mixed solution is 2.0 - 6.0.

10. A method for preparing a carbataxel composition for injection comprising the following steps:
(1) weighing the cyclodextrin, adding the solubilizer and water, adding the polyvidone and the additive after stirring to dissolve, stirring to dissolve, and then adding cabazitaxel and filling with inert gas for protection, continuing to stir for 30 - 240min after stirring to dissolve, and obtaining an uniform mixed solution;
(2) taking samples to determine pH value and concentration, after testified to be qualified, filtering through 0.2 µm polytetrafluoroethylene (PTFE) membrane, sub-packing in vials, filling nitrogen, pluging, sealing and labeling;
preferably, in the step (1), the pH value of the uniform mixed solution is 2.0 - 6.0.

11. A method for treating tumors, comprising administering the composition according to any one of claims 1 to 8 or the composition prepared by the method according to claim 9 or 10 to a patient in need; preferably, the tumor is prostate cancer.
